# EUROPEAN PATENT SPECIFICATION

(11) **EP 3 484 373 B1**
(45) Date of publication and mention of the grant of the patent: **07.04.2021**
(21) Application number: 16757341.9
(22) Date of filing: 16.07.2016
(51) Int. Cl.: A61B 10/02, A61B 5/053

(54) **A BIOPSY GUN, A BIOPSY SAMPLE COLLECTING SYSTEM AND A METHOD FOR CONNECTING AT LEAST ONE NEEDLE ELECTRODE OF A BIOPSY NEEDLE TO A CONNECTOR AND/OR TO A MEASUREMENT CABLE IN A BIOPSY GUN**
BIOPSIEPISTOLE, BIOPSIEPROBENAHMESYSTEM UND VERFAHREN ZUR VERBINDUNG VON MINDESTENS EINER NADELELEKTRODE EINER BIOPSIENADEL MIT EINEM VERBINDER UND/ODER MIT EINEM MESSKABEL IN EINER BIOPSIEPISTOLE
PISTOLET DE BIOPSIE, SYSTÈME DE PRÉLÈVEMENT D'ÉCHANTILLON DE BIOPSIE ET PROCÉDÉ DE CONNEXION D'AU MOINS UNE ÉLECTRODE D'AIGUILLE D'UNE AIGUILLE DE BIOPSIE À UN CONNECTEUR ET/OU À UN CÂBLE DE MESURE DANS UN PISTOLET DE BIOPSIE

(43) Date of publication of application: 22.05.2019
(73) Proprietor: Injeq Oy, 33520 Tampere (FI)
(72) Inventor: KRONSTRÖM, Kai, 02200 Espoo (FI); AHONEN, Petri, 33720 Tampere (FI); ELOMAA, Timo, 33960 Pirkkala (FI); HALONEN, Sanna, 33720 Tampere (FI); KARI, Juho, 33270 Tampere (FI)
(74) Representative: Genip Oy
(86) International application number: PCT/IB2016/054253
(87) International publication number: WO 2018/015782

(56) References cited:
- WO-A1-00/56224
- US-A1- 2001 002 250
- US-A1- 2002 019 596
- US-A1- 2015 038 872
- US-B1- 6 277 083

## Description

### Technical field

The present invention relates to medical technology, more particularly to core needle biopsy devices and biopsy needles.

### Background art

Accurate diagnosis and determination of the stage of a disease are enabled by tissue samples, biopsies. Biopsy samples are involved in cancer detection, follow-up of treatment, estimation of disease severity and prognosis. For example, prostate biopsies are utilized for confirming suspected prostate cancer, liver biopsies for determining the progression of fibrosis and cirrhosis and response of treatment for hepatitis and renal biopsies are for different kidney diseases and dysfunctions as well as for transplantation follow-up.

Biopsies have an extremely important role since treatment decisions are often based on them. An extracted tissue sample is small in order to be minimally invasive and as a result, the biopsy sample covers only a small portion of the total volume of the sampled organ. Because of heterogeneity and variation within the organ, the actual target may get missed. In liver tissue, for example, lesions of hepatitis are unevenly distributed which may lead to sampling error and misdiagnosis [1]. Standard prostate biopsy procedure includes several biopsies taken systematically from different sites of the organ, but is still insufficient. In fact, risk in prostate cancer for false negative detection is high: In study of Sonn et al. [2] 38% of Gleason score higher than 7 was not detected with systematic biopsy.

Electrical properties of tissues differ from each other enabling tissue discrimination by bioimpedance spectroscopy. When placing measurement electrode inside the injection needle, bioimpedance can be utilized in needle guidance [3]. The first bioimpedance probe needles were thick, but Kari et al. [4] adapted the bioimpedance measurement in bipolar fashion to standard commercial hypodermic needle and used it with a real time classifier for tissue identification.

In addition to different tissue types, cancerous and benign tissues could be differentiated: For example, malign tumors in breast, lung, prostate and kidney have shown to cause significant changes to the electrical properties of tissue [5] - [10]. Therefore the bioimpedance based targeted biopsy is expected to enable more accurate tissue sampling.

One kind of core type biopsy instrument has two nested needles, an outer and an inner needle with a biopsy cavity for picking a tissue sample. The biopsy needle is in loaded state when set to the target site and then fired forward for sample intake. For example, in prostate cancer the biopsy sample covers a volume 1.5 cm from the needle tip [11].

Mishra et al. [12] discloses a real time bioimpedance measuring biopsy needle. It measures the impedance between the inner needle tip and outer needle tip.

Published patent application US2015/038872 A1 discloses a multi electrode electrical impedance biopsy sampling device. Published patent application US2002/019596 A1 discloses an electrosurgical cutting device for recovering volumes of tissue. Published patent application US 2001/002250 A1 discloses a method for determining a biopsy needle location by means of radiation energy detector. Published patent application WO 00/56224 A1 discloses an apparatus for performing myocardial revascularization with a catheter. Patent US 6277083 discloses a an apparatus for invasively recovering tissue.

### Objective of the invention

An objective of the invention is to improve the reliability of core needle biopsy. This objective can be achieved with the biopsy gun according to the independent claim 1, with the biopsy sample collecting system according to the independent claim 11, and with the method according to parallel independent claim 13. The dependent claims describe various advantageous aspects of the biopsy gun, of the biopsy sampling system and of the method.

### Advantages of the invention

The biopsy gun comprises a biopsy tool handle. A biopsy needle comprising a first needle, at least one another needle movable in relation to the first needle, and a biopsy cavity has been attached to the biopsy tool handle or is attachable to the biopsy tool handle.

The biopsy tool handle comprises a firing part or is attachable to the same. The firing part is configured to cause a relative movement of the first needle and of the at least one another needle in such a way that their relative movement causes a biopsy sample to be captured in the biopsy cavity.

Furthermore, the biopsy gun comprises at least one resilient contact, such as a contact spring, for creating a galvanic contact with a respective biopsy needle electrode, connected to a connector or to a measurement cable.

The biopsy gun has been configured to:
i) galvanically connect the at least one electrode to the connector or to the measurement cable when the firing part is loaded,
   and/or
ii) to disconnect the galvanic connection between the at least one electrode and the connector or the measurement cable when the firing part is fired.

With the first aspect of the biopsy gun, the reliability of core needle biopsy can be improved, since the biopsy gun now requires that the firing part must be loaded before the at least one electrode will be galvanically connected to the connector and/or to the measurement cable. This helps to prevent a situation where the biopsy gun were loaded only after the positioning of the distal end of the biopsy needle in tissue. Loading the firing part of the biopsy gun after positioning could easily lead to the distal end of the biopsy needle to get displaced which would be prone to cause the biopsy sample becoming collected not from the intended position defined with the bioimpedance measurement.

With the second aspect of the biopsy gun, the reliability of core needle biopsy can be improved, since the biopsy gun now ensures that the at least one electrode is galvanically disconnected from the connector and/or from the measurement cable when the firing part is fired. This helps to prevent a situation where the bioimpedance were measured still after firing the biopsy gun. Measuring bioimpedance after firing the biopsy gun would reduce the reliability of tissue characterization since tissue impedance measurement characteristics inevitably changes during to firing due to a relative movement or relative movements between the bioimpedance electrodes.

If the at least one resilient contact is stationary and the biopsy gun is arranged to move the first needle and the at least one another needle in relation to each other by displacing a respective anchor or anchors, the making/removing of the galvanic connection can be carried out more predictably than displacing the at least one resilient contact.

The galvanic connection between the at least one needle electrode and the connector may be made at the anchor or the anchors, respectively. Preferably, the anchor comprises an anchor contact and the respective resilient contact is suited to touch the anchor contact when the firing part is loaded and/or not to touch the anchor contact after firing of the firing part.

The galvanic connection between the at least one needle electrode and the connector can be made at the first needle. This is particularly advantageous if the first needle is the inner needle of a core biopsy needle in which the outer needle cuts the biopsy sample into the biopsy cavity like in a side-notch biopsy needle, or if the first needle is the stylet of a co-axial pinch type core biopsy needle, in which the inner coring cannula surrounds the biopsy sample and the outer cannula with pincer cuts the biopsy sample into the biopsy cavity.

Preferably, the at least one resilient contact is arranged at a biopsy gun lid. In this manner, a bioimpedance measurement without a biopsy needle installed can be prevented more reliably since the biopsy gun lid will be opened with the biopsy needle is removed. Likewise, a bioimpedance measurement with a biopsy needle installed improperly can be avoided.

Preferably, the at least one resilient contact is located in a receiving compartment for a biopsy needle. This enables the simplification of the mechanical structure that is used to make/disconnect the galvanic connection.

The biopsy needle comprises a first needle, a second needle, and a biopsy cavity. The first needle and the second needle have a distal end having a form suitable for cutting tissue. Furthermore, the first needle and the second needle have been dimensioned such that, when the biopsy needle is introduced in tissue, a biopsy sample will be captured in the biopsy cavity by at least one relative movement between the first needle and the second needle. A wire electrode functions as a measuring electrode. The wire electrode extends outside from the inner needle to have a larger contact area for making a galvanic contact with a resilient contact of the biopsy gun.

The biopsy needle enables improving the reliability of core needle biopsy when used together with the biopsy gun, for reasons already explained.

Preferably, the wire electrode continues to or is galvanically connected to an anchor contact. A better quality of the galvanic connection can be ensured if the contacting between the wire electrode and the resilient contact is made at the anchor contact.

The biopsy sample collecting system comprises the biopsy gun and a biopsy needle having at least one needle electrode. The connector or the measurement cable is connected to an impedance measurement device suitable for measuring bioimpedance. The biopsy sample collecting system enables improving the reliability of core needle biopsy, for reasons already explained.

In the biopsy sample collecting system, the impedance measurement device may further be connected to at least one electrode located outside of the biopsy needle such that the at least one electrode is remote to the at least one needle electrode. Preferably, the at least one electrode is adhered to the skin of a person. This enables bioimpedance-guided biopsy sampling where the measurement of the bioimpedance is carried out in a monopolar fashion.

In the method for connecting at least one needle electrode of a biopsy needle to a connector or to a measurement cable in a biopsy gun
i) the at least one needle electrode is galvanically connected to the connector or to the measurement cable when the firing part is loaded,
   and/or
ii) the galvanic connection between the at least one needle electrode and the connector or the measurement cable is disconnected when the firing part is fired.

The method enables improving the reliability of core needle biopsy when used together with the biopsy gun, for reasons already explained.

Preferably, the connecting/disconnecting is carried out by displacing at least one of the anchors of the biopsy needle. This improves ensuring the quality of the galvanic connection.

The connecting and/or disconnecting may be carried out by moving the wire electrode to/from a resilient contact that protrudes from the biopsy gun lid to a receiving compartment. This helps to avoid carrying out the bioimpedance measurement when the biopsy gun is not loaded. The displacement may be or include at least one lateral and/or rotational movement.

The galvanic connection can be made or disconnected in response to a relative movement of the first needle and the second needle. The relative movement may be or include at least one lateral and/or rotational movement.

### List of drawings

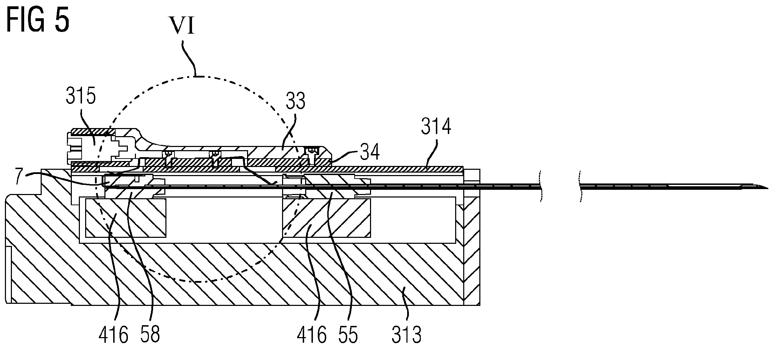

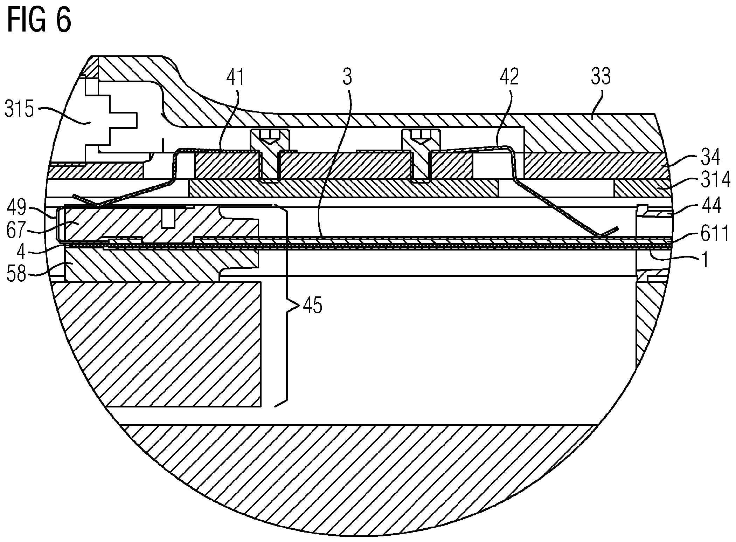

The invention is explained in more detail in the following by way of the exemplary embodiments shown in the attached drawings in FIG 1 to 6. In the drawings:
- FIG 1: illustrates a side-notch biopsy needle, the biopsy cavity exposed (after advancing of the inner needle but before advancing of the outer needle);
- FIG 2: illustrates the section II-II of the side-notch biopsy needle;
- FIG 3: illustrates a biopsy gun with lid closed;
- FIG 4: illustrates the biopsy gun of FIG 3 with lid open;
- FIG 5: illustrates section V-V of the biopsy gun with lid closed; and
- FIG 6: illustrates the detail VI.

Similar parts are marked with identical reference numbers in all FIG.

### Detailed description

The biopsy gun, the biopsy needle and the method are in the following described in detail with reference to embodiments that involve the use of a conventional co-axial pinch type core biopsy needle that has been improved by adding measuring electrodes. However, the biopsy gun and the biopsy needle can respectively be implemented in the framework of a tri-axial core, cut and capture cannula system, or of a rotating biopsy needle. In the tri-axial core, cut and capture cannula system, for example, the hollow distal end of inner coring cannula is fired to surround the biopsy sample and then the outer cannula with a pincer cuts the biopsy sample into the biopsy cavity.

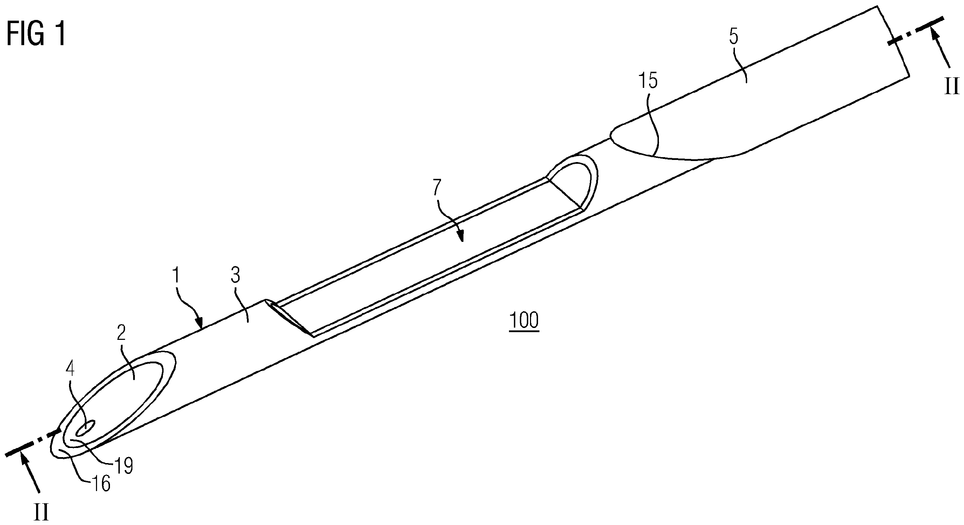

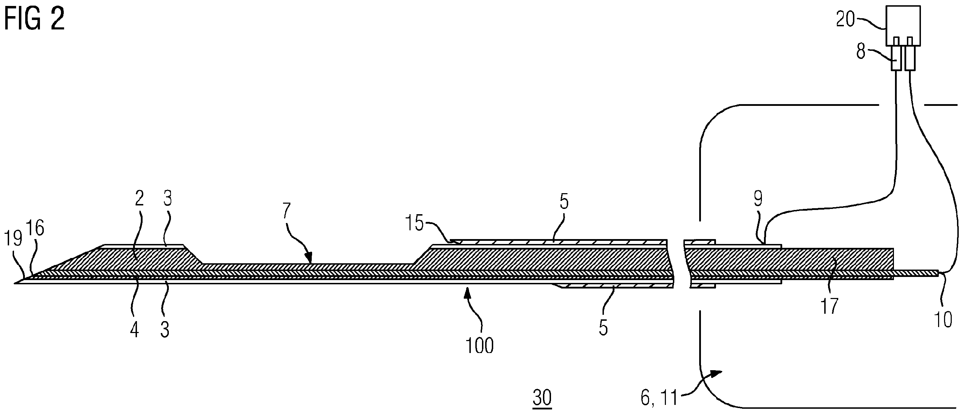

Referring to FIG 1 and 2, a biopsy needle (side-notch biopsy needle 100) for core needle biopsy comprises an outer needle 5 with a distal end 15, an inner needle 1 that has a sharpened distal end 16, and at least one biopsy cavity 7. In the co-axial pinch type core biopsy needle, the biopsy cavity may be located at distance from the sharpened distal end 16, but this is not necessary, especially if the biopsy needle has been implemented differently.

The outer needle 5 and the inner needle 1 are configured so that the inner needle 1 is accommodated inside the cannula 5 and the inner needle 1 and the cannula 5 are movable with regard to each other. The inner needle 1 comprises a shell 3 that is a measuring electrode and a polymer filled core 2 that insulates the shell 3 galvanically from the wire electrode 4. Core 2 can also comprise or consist of metal, polymer alloy/epoxy or/and composite material polymer and glass fiber, for example. The core 2 should preferably in all cases have a high Young's modulus to manufacture a rigid inner needle 1. The cannula 5 is preferably made of steel or other material that can be enabling manufacture of the sharpened cutting edge to the distal end 15.

Shell 3 and wire electrode 4 function as two measuring electrodes for measuring a bioimpendance spectra. Both are located before the biopsy cavity 7 as seen from the distal end 16, while at least one of the measuring electrodes is located along the distance and/or essentially only in the distal end 16 of the inner needle 1 preferably in such a manner that the measuring electrodes define measuring volume that is preferably localized around the distal end 16 of the inner needle 1, and most preferably focused on the beveled needle facet 19.

The length and location of the biopsy cavity 7 along the inner needle 1 are preferably selected in such a manner that the firing by a firing part of a biopsy gun 30 moves the biopsy cavity 7 into the measuring volume so that at least some, preferably most of the measuring volume will be essentially covered by the biopsy cavity 7.

The biopsy gun 30 further comprises an electrical connection 8 to a measurement device 20, an electrical coupling 9 to the inner needle 1 and an electrical coupling 10 to the electrode wire 4. The connecting is preferably done at the proximal end 17 of the biopsy needle 100.

The arrangement, number and structure of the measuring electrodes may be chosen differently, especially if the biopsy needle is not a conventional side-notch core biopsy needle.

To summarize, the biopsy needle 100 that is suitable for a biopsy gun 30, comprises a first needle (preferably the inner needle 1), a second needle (preferably the outer needle 5), and a biopsy cavity 7. The first needle (inner needle 1) and the second needle (outer needle 5) have both a distal end having a form suitable for cutting tissue.

The first needle (inner needle 1) and the second needle (outer needle 5) are arranged such that, when the biopsy needle 100 is introduced in tissue, a biopsy sample will be captured in the biopsy cavity 7 by at least one relative movement between the first needle (inner needle 1) and the second needle (outer needle 5).

The first needle (inner needle 1) functions as one measuring electrode 3 and the wire electrode 4 functioning as another measuring electrode. The wire electrode 4 extends outside from the inner needle 1 to have a larger contact area for making a galvanic contact with a resilient contact 41, 42 of the biopsy gun 30.

Preferably, the wire electrode 4 continues to or is galvanically connected to an anchor contact 49.

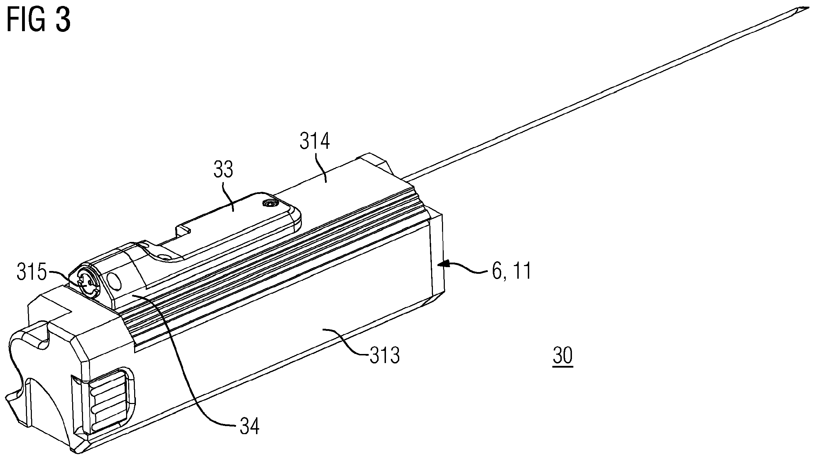

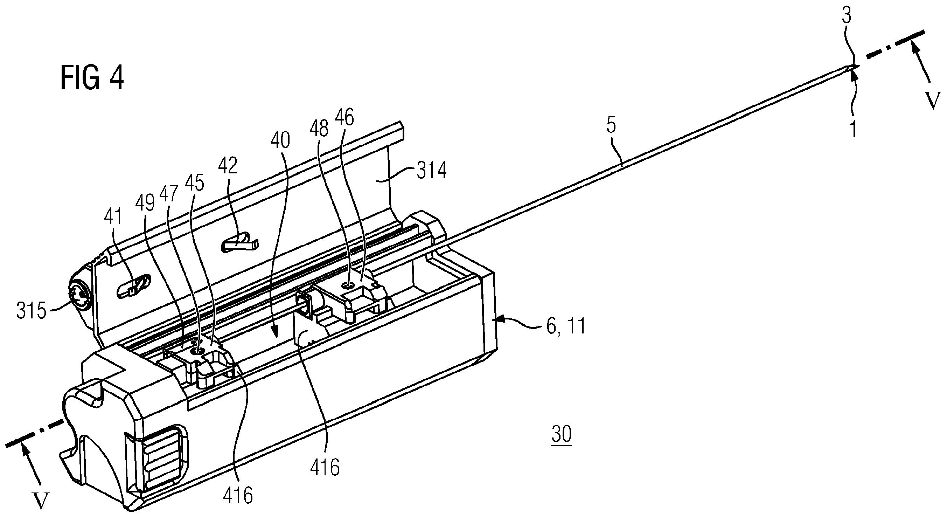

Going now to FIG 3 and 4, the biopsy gun 30 comprises a biopsy handle 6 with a firing part 11. The gun base 313 has a lid 314 that is preferably openable. The biopsy needle 100 has been attached to the biopsy gun 30, or is attachable to the same after opening the lid 314, after which the lid 314 is closed. The biopsy gun 30 further comprises a firing part 11, which is configured to fire the first needle (inner needle 1) outwards from the second needle (outer needle 5) and to make the second needle (outer needle 5) to follow the first needle (inner needle 1) in such a way that at least the first needle (inner needle 1) moves a punch length forward and the second needle (outer needle 5) follows it by moving from behind of the biopsy cavity 7 past the biopsy cavity 7 in order to capture a tissue sample. As explained above, the biopsy needle 100 is configured to pick a tissue sample preferably from the volume which spatially covers some of and preferably most of the measuring volume, into the biopsy cavity 7.

A biopsy needle 100 comprising a first needle (inner needle 1), at least one second needle (outer needle 5) movable in relation to the first needle (inner needle 1), and a biopsy cavity 7 has been attached to the biopsy tool handle 6, or is attachable to the same.

The biopsy tool handle 6 comprises or is attached to a firing part 11, which is configured to cause a relative movement of the first needle (inner needle 1) and of the at least one second needle (outer needle 5) in such a way that their relative movement causes a biopsy sample to be captured into the biopsy cavity 7.

The biopsy tool handle 6 further comprises at least two resilient contacts 41, 42, such as contact springs, each for creating a galvanic contact with a respective biopsy needle electrode 4, 3, both connected to a connector 315 and/or to a measurement cable.

The biopsy gun 30 has been configured to galvanically connect at least one of the electrodes 4, 3 to the connector 315 and/or to the measurement cable when the firing part 11 is loaded. Alternatively or in addition, the biopsy gun has been configured to disconnect the galvanic connection between at least one of the electrodes 4, 3 and the connector 315 and/or the measurement cable when the firing part 11 is fired.

The resilient contacts 41, 42 are preferably stationary and the biopsy gun 30 is arranged to move the first needle (inner needle 1) and the at least one second needle (outer needle 5) in relation to each other by displacing a respective anchor or anchors 45, 46 . The galvanic connection between at least one of the needle electrodes 4, 3 and the connector 315 may be made at each one of said anchors 45, 46, respectively.

The anchor 45 preferably comprises an anchor contact 49 and the respective resilient contact 41 is suited to touch the anchor contact 49 when the firing part 11 is loaded and/or not to touch the anchor contact 49 after firing of the firing part 11.

The galvanic connection between at least one of the needle electrodes 3, 4 and the connector 315 can be made at the inner needle 1.

Optimally, at least one (but preferably both or all) of the at least two resilient contacts 41, 42 is arranged at a biopsy gun lid 314. In addition, the resilient contact or contacts 41, 42 may be located in a receiving compartment 40 for the biopsy needle 100.

In the method for connecting at least two electrodes 3, 4 of a biopsy needle 100 to a connector 315 and/or to a measurement cable in a biopsy gun 30, at least one of the electrodes 4, 3 is galvanically connected to the connector 315 and/or to the measurement cable when the firing part 11 is loaded. Alternatively or in addition, the galvanic connection between at least one of the needle electrodes 4, 3 and the connector 315 and/or the measurement cable is disconnected when the firing part 11 is fired.

Preferably, the connecting and/or disconnecting is carried out by displacing at least one of the anchors 45, 46 of the biopsy needle 100.

The connecting/disconnecting may be carried out by moving the wire electrode 4 to/from a resilient contact 41, 42 that protrudes from the biopsy gun lid 314 to a receiving compartment 40. The moving may be or include at least one lateral and/or rotational movement.

The galvanic connection can be made or disconnected in response to a relative movement of the first needle (inner needle 1) and the second needle (outer needle 5). The relative movement may be or include at least one lateral and/or rotational movement.

It is obvious to the skilled person that, along with the technical progress, the basic idea of the invention can be implemented in many ways. The invention and its embodiments are thus not limited to the examples described above but they may vary within the contents of patent claims and their legal equivalents.

The biopsy gun, the biopsy needle, the biopsy sample collecting system and the method have been described above in the context of a biopsy needle 100 having two needle electrodes: as one needle electrode is the wire electrode 4 and as the other measuring electrode 3 is the shell of the inner needle 1.

However, instead of having the biopsy needle 100 with two needle electrodes, the biopsy needle can be implemented with more (such as, with three, four, five, six) needle electrodes, or with less electrodes, namely, with one needle electrode only.

If there is only one needle electrode, it can be either the wire electrode, or the shell of the inner needle 1, or, if the biopsy needle is being implemented differently, in any suitable part of the biopsy needle.

The biopsy needle with only one needle electrode is useable especially in order to carry out the bioimpedance measurementguided biopsy sampling, where the bioimpedance measurement is carried out in a monopolar fashion in which one electrode is dominant in a nonsymmetrical system of two or more electrodes. Preferably, as the non-dominant electrode will be an electrode tag adhered to the skin of the patient. The non-dominant electrode normally needs to be large enough to ensure that the needle electrode dominates the bioimpedance measurement.

The biopsy needle with only one needle electrode enables a simpler construction of the biopsy needle, in contrast to a biopsy needle with at least two needle electrodes that can be used to carry out bioimpedance measurement guided biopsy sampling, where the measurement of the bioimpedance is carried out in a bipolar fashion, where two equal electrodes are in a symmetrical or in an almost symmetrical system. With symmetry we mean that both electrodes equally influence the measured impedance.

### References:

[1] Ratziu, V., Charlotte, F., Heurtier, A., Gombert, S., Giral, P., Bruckert, E., ... & LIDO Study Group "Sampling variability of liver biopsy in nonalcoholic fatty liver disease." Gastroenterology 128.7 (2005): 1898-1906.
[2] Sonn, G. A., Natarajan, S., Margolis, D. J., MacAiran, M., Lieu, P., Huang, J., ... & Marks, L. S. "Targeted biopsy in the detection of prostate cancer using an office based magnetic resonance ultrasound fusion device." The Journal of urology 189.1 (2013): 86-92.
[3] Kalvoy, H., Frich, L., Grimnes, S., Martinsen,. G., Hol, P. K., & Stubhaug, A. "Impedance-based tissue discrimination for needle guidance." Physiological measurement 30.2 (2009): 129.
[4] Kari J, Annala K, Annus P, Seppa V-P and Kronström K. "A thin needle with bio-impedance measuring probe: tissue recognition performance assessed in in vivo animal study." Available at www.injeq.com, BRC 3.0, 2015, cited in 12.5.2015.
[5] Kimura, S., Morimoto, T., Uyama, T., Monden, Y., Kinouchi, Y., & Iritani, T. "Application of electrical impedance analysis for diagnosis of a pulmonary mass." Chest Journal 105.6 (1994): 1679-1682.
[6] Morimoto, T., Kimura, S., Konishi, Y., Komaki, K., Uyama, T., Monden, Y., ... & Iritani, D. T. "A study of the electrical bio-impedance of tumors." Investigative Surgery 6.1 (1993): 25-32.
[7] Jossinet, J. "The impedivity of freshly excised human breast tissue." Physiological measurement 19.1 (1998): 61.
[8] Halter, R. J., Schned, A., Heaney, J., Hartov, A., & Paulsen, K. D. "Electrical properties of prostatic tissues: I. Single frequency admittivity properties." the Journal of Urology 182.4 (2009): 1600-1607.
[9] Halter, R. J., Schned, A., Heaney, J., Hartov, A., & Paulsen, K. D. "Electrical properties of prostatic tissues: II. Spectral admittivity properties." The Journal of urology 182.4 (2009): 1608-1613.
[10] Inagaki, T., Bhayani, S. B., Allaf, M. E., Ong, A. M., Rha, K. H., Petresior, D., ... & Kavoussi, L. R. "Tumor capacitance: electrical measurements of renal neoplasia." The Journal of urology 172.2 (2004): 454-457.
[11] Patel, Amit R., and J. Stephen Jones. "The prostate needle biopsy gun: busting a myth." The Journal of urology 178.2 (2007): 683-685.
[12] Mishra, V., Bouayad, H., Schned, A., Hartov, A., Heaney, J., & Halter, R. J.. "A real-time electrical impedance sensing biopsy needle." Biomedical Engineering, IEEE Transactions on 59.12 (2012): 3327-3336.

### List of reference numbers used:

- 1: inner needle
- 2: core
- 3: measuring electrode
- 4: wire electrode
- 5: outer needle
- 6: biopsy gun handle
- 7: biopsy cavity
- 8: electrical connection
- 9: electrical coupling
- 10: electrical coupling
- 11: firing part
- 15: distal end
- 16: distal end
- 17: proximal end
- 19: facet
- 20: measurement device
- 30: biopsy gun
- 33: connector hub cover
- 34: connector hub base
- 40: receiving compartment
- 41: contact spring
- 42: contact spring
- 45: anchor
- 46: anchor
- 47: pin
- 48: pin
- 49: anchor contact
- 416: biopsy gun slide
- 58: lower inner needle anchor
- 67: upper inner needle anchor
- 100: side-notch biopsy needle
- 313: biopsy gun base
- 314: biopsy gun lid
- 315: coaxial socket connector for cable
- 611: insulation

## Claims

1. A biopsy gun (30), comprising:
- a biopsy tool handle (6), to which a biopsy needle (100) comprising a first needle (1), at least one another needle (5) movable in relation to the first needle (1), and a biopsy cavity (7), has been attached, the biopsy tool handle (6) comprising or attached to a firing part (11), which is configured to cause a relative movement of the first needle (1) and of the at least one another needle (5) in such a way that their relative movement causes a biopsy sample to be captured in the biopsy cavity (7);
wherein the biopsy gun (30) further comprises at least one resilient contact (41, 42), such as at least one contact spring, for creating a galvanic contact with a respective biopsy needle electrode (4, 3), and connected to a connector (315) or to a measurement cable;
and wherein the biopsy gun (30) has been configured to:
i) galvanically connect the least one electrode (4, 3) to the connector (315) or to the measurement cable when the firing part (11) is loaded,
and/or
ii) to disconnect the galvanic connection between the at least one electrode (4, 3) and the connector (315) or the measurement cable when the firing part (11) is fired.

2. The biopsy gun (30) according to claim 1, wherein: the at least one resilient contact (41, 42) is stationary and the biopsy gun (30) is arranged to move the first needle (1) and the at least one another needle (5) in relation to each other by displacing a respective anchor (45, 46).

3. The biopsy gun (30) according to claim 2, **wherein:** the galvanic connection between the at least one needle electrode (4, 3) and the connector (315) is made at the anchor (45, 46) or at each of the anchors, respectively.

4. The biopsy gun (30) according to claim 3, **wherein:** the anchor (45) comprises an anchor contact (49) and the respective resilient contact (41) is suited to touch the anchor contact (49) when the firing part (11) is loaded and/or not to touch the anchor contact (49) after firing of the firing part (11).

5. The biopsy gun (30) according to any one of the preceding claims, **wherein:** the galvanic connection between the least one needle electrode (4, 3) and the connector (315) is made at the first needle (1).

6. The biopsy gun (30) according to any one of the preceding claims, **wherein:** the at least one resilient contact (41, 42) is arranged at a biopsy gun lid (314).

7. The biopsy gun (30) according to any one of the preceding claims, **wherein:** the at least one resilient contact (41, 42) is located in a receiving compartment (40) for a biopsy needle (100) .

8. The biopsy gun (30) according to any one of the preceding claims, **wherein:** the biopsy needle (1) has at least two needle electrodes (4, 3),
- of which at least one or two are galvanically connected to the connector (315) or to the measurement cable when the firing part (11) is loaded,
and/or
of which the galvanic connection to the connector (315) or the measurement cable is disconnected when the firing part (11) is fired.

9. A biopsy gun (30) according to any one of the preceding claims, **wherein:**
- the first needle (1) and the at least one other needle (5) have a distal end having a form suitable for cutting tissue;
- the biopsy needle electrode (4) comprises a wire electrode (4) functioning as a measuring electrode, the wire electrode (4) extending outside from the first needle (1) to have an enlarged contact area for making a galvanic contact with a resilient contact (41, 42) of the biopsy gun (30).

10. The biopsy gun (30) according to claim 9, **wherein:** the wire electrode (4) continues to or is galvanically connected to an anchor contact (49).

11. A biopsy sample collecting system, **comprising:** a biopsy gun (30) according to any one of the preceding claims 1 to 10, and wherein the connector (315) or the measurement cable is connected to an impedance measurement device suitable for measuring bioimpedance.

12. The biopsy sample collecting system according to claim 11, **wherein:** the impedance measurement device is further connected to at least one electrode located outside of the biopsy needle (100) such that the at least one electrode is remote to the at least one needle electrode (3, 4). Preferably, the at least one electrode is adhered to the skin of a person.

13. A method for connecting at least one needle electrode (4, 5) of a biopsy needle (100) to a connector (315) or to a measurement cable in a biopsy gun (30) according to any one of the preceding claims 1 to 10, the method comprising the **step of:**
i) galvanically connecting the at least one needle electrode (4, 3) to the connector (315) or to the measurement cable when the firing part (11) is loaded,
and/or
ii) disconnecting the galvanic connection between the at least one needle electrode (4, 3) and the connector (315) or the measurement cable when the firing part (11) is fired.

14. The method according to claim 13, **wherein:** the connecting and/or disconnecting is carried out by displacing least one anchor (45, 46) of the biopsy needle (100).

15. The method according to claim 13 or 14, **wherein:** the connecting/disconnecting is carried out by moving the wire electrode (4) to/from a resilient contact (41, 42) that protrudes from the biopsy gun lid (314) to a receiving compartment (40).

16. The method according to claim 15, **wherein:** the moving is or includes at least one lateral and/or rotational movement.

17. The method according to any one of claims 13 to 16, **wherein:** the galvanic connection is made or disconnected in response to a relative movement of the first needle (1) and the second needle (5) .

18. The method according to claim 17, **wherein:** the relative movement is or includes at least one lateral and/or rotational movement.

19. The method according to any one of the claims 13 to 18, **wherein:** the biopsy needle (1) has at least two needle electrodes (4, 3),
- of which at least one or two are galvanically connected to the connector (315) or to the measurement cable when the firing part (11) is loaded,
and/or
of which the galvanic connection to the connector (315) or the measurement cable is disconnected when the firing part (11) is fired.

20. The method according to any one of the claims 13 to 19, **wherein:** the connector (315) or the measurement cable is connected to an impedance measurement device suitable for measuring bioimpedance.

21. The method according to claim 20, **wherein:** the measurement device is further connected to at least one remote electrode, such as an electrode adhered to the skin of a person.

## Patentansprüche

1. Biopsiepistole (30), die folgendes umfasst:
- einen Biopsieinstrumentengriff (6), an dem eine Biopsienadel (100) befestigt wurde, die eine erste Nadel (1), mindestens eine andere Nadel (5), die im Bezug auf die erste Nadel (1) bewegt werden kann, und einen Biopsie-Hohlraum (7) umfasst, der Biopsieinstrumentengriff (6) einen Abschussteil (11) umfasst oder daran befestigt ist, der für das Bewirken einer relativen Bewegung der ersten Nadel (1) und der mindestens einen anderen Nadel (5) auf eine solche Weise ausgelegt ist, dass ihre relative Bewegung das Fangen der Biopsieprobe im Biopsie-Hohlraum (7)bewirkt;
worin
die Biopsiepistole (30) außerdem mindestens einen gefederten Kontakt (41, 42) umfasst, wie mindestens eine Kontaktfeder, um einen galvanischen Kontakt mit einer betreffenden Biopsienadelelektrode (4, 3) herzustellen und verbunden mit einem Konnektor (315) oder mit einem Messkabel;
und worin die Bieopsiepistole (30) ausgelegt ist, um:
i) die mindestens eine Elektrode (4, 3) mit dem Konnektor (315) oder mit dem Messkabel galvanisch zu verbinden, wenn der Abschussteil (11) geladen ist,
und/oder
ii) die galvanische Verbindung zwischen der mindestens einen Elektrode (4, 3) und dem Konnektor (315) oder dem Messkabel zu unterbrechen, wenn der Abschussteil (11) abgefeuert ist.

2. Biopsiepistole (30) nach Anspruch 1, **worin:** der mindestens eine federnde Kontakt (41, 42), feststehend ist und die Biopsiepistole (30) angeordnet ist, um die erste Nadel (1) und die mindestens eine andere Nadel (5) im Bezug aufeinander durch Verschieben eines betreffenden Ankers (45, 46) zu bewegen.

3. Die Biopsiepistole (30) nach Anspruch 2, **worin:** die galvanische Verbindung zwischen der mindestens einen Nadelelektrode (4, 3) und dem Konnektor (315) jeweils am Anker (45, 46) oder an jedem der Anker zustande kommt.

4. Biopsiepistole (30) nach Anspruch 3, **worin:** der Anker (45) einen Ankerkontakt (49) umfasst und der jeweilige gefederte Kontakt (41) geeignet ist, den Ankerkontakt (49) zu berühren, wenn der Abschussteil (11) geladen ist und/oder den Ankerkontakt (49) nicht zu berühren nachdem der Abschussteil (11) abgefeuert ist.

5. Biopsiepistole (30) nach einem der vorhergehenden Ansprüche, **worin:** die galvanische Verbindung zwischen der mindestens einen Nadelelektrode (4, 3) und dem Konnektor (315) an der ersten Nadel (1) hergestellt ist.

6. Biopsiepistole (30) nach einem der vorhergehenden Ansprüche, **worin:** der mindestens eine gefederte Kontakt (41, 42) an einer Biopsiepistolenabdeckung (314) angeordnet ist.

7. Biopsiepistole (30) nach einem der vorhergehenden Ansprüche, **worin:** der mindestens eine gefederte Kontakt (41, 42) in einem Aufnahmefach (40) für eine Biopsienadel (100) sitzt.

8. Biopsienadel (30) nach einem der vorhergehenden Ansprüche, **worin:** die Biopsienadel (1) mindestens zwei Nadelelektroden (4, 3) aufweist,
- von denen mindestens eine oder zwei galvanisch mit dem Konnektor (315) oder dem Messkabel verbunden ist/sind, wenn der Abschussteil (11) geladen ist,
und/oder
von denen die galvanische Verbindung zum Konnektor (315) oder zum Messkabel unterbrochen wird, wenn der Abschussteil (11) abgefeuert ist.

9. Biopsiepistole (30) nach einem der vorhergehenden Ansprüche, **worin:**
- die erste Nadel (1) und die mindestens eine andere Nadel (5) ein distales Ende mit einer Form aufweisen, die zum Schneiden von Gewebe geeignet ist;
- die Biopsienadelelektrode (4) eine Drahtelektrode (4) umfasst, die als Messelektrode fungiert, die Drahtelektrode (4) sich von der ersten Nadel (1) aus nach außen erstreckt, um einen vergrößerten Kontaktbereich zur Herstellung eines galvanischen Kontaktes mit einem gefederten Kontakt (41, 42) der Biopsiepistole (30) zu haben.

10. Biopsiepistole (30) nach Anspruch 9, **worin:** die Drahtelektrode (4) bis zu einem Ankerkontakt (49) geht oder mit einem solchen galvanisch verbunden ist.

11. Biopsieprobenentnahmesystem, **das folgendes umfasst:** eine Biopsiepistole (30) nach einem der vorhergehenden Ansprüche 1 bis 10, und worin der Konnektor (315) oder das Messkabel mit einem Impedanzmessgerät verbunden ist, das zur Messung von Bioimpedanz geeignet ist.

12. Biopsieprobenentnahmesystem nach Anspruch 11, **worin:** das Impedanzmessgerät außerdem mit mindestens einer Elektrode verbunden ist, die derart außerhalb der Biopsienadel (100) sitzt, dass die mindestens eine Elektrode von der mindestens einen Nadelelektrode (3, 4) entfernt ist. Vorzugsweise ist die mindestens eine Elektrode auf der Haut einer Person befestigt.

13. Verfahren zum Verbinden mindestens einer Nadelelektrode (4, 5) einer Biopsienadel (100) mit einem Konnektor (315) oder mit einem Messkabel in einer Biopsiepistole (30) nach einem der vorhergehenden Ansprüche 1 bis 10, **wobei das Verfahren den folgenden Schritt umfasst:**
i) galvanisches Verbinden der mindestens einen Nadelelektrode (4, 3) mit dem Konnektor (315) oder mit dem Messkabel, wenn der Abschussteil (11) geladen wird,
und/oder
ii) unterbrechen der galvanischen Verbindung zwischen der mindestens einen Nadelelektrode (4, 3) und dem Konnektor (315) oder dem Messkabel, wenn der Abschussteil (11) abgefeuert wird.

14. Verfahren nach Anspruch 13, **worin:** die Verbindung oder Unterbrechung durch Verschieben mindestens eines Ankers (45, 46) der Biopsienadel (100) durchgeführt wird.

15. Verfahren nach Anspruch 13 oder 14, **worin:** die Verbindung / Unterbrechung durch Bewegen der Drahtelektrode (4) hin / weg von einem gefederten Kontakt (41, 42) durchgeführt wird, der aus der Biopsiepistolenabdeckung (314) heraus-, in ein Aufnahmefach (40) hineinragt.

16. Verfahren nach Anspruch 15, **worin:** die Bewegung mindestens eine laterale Bewegung und/oder eine Rotationsbewegung ist oder einschließt.

17. Verfahren nach einem der Ansprüche 13 bis 16, **worin:** die galvanische Verbindung als Antwort auf eine relative Bewegung der ersten Nadel (1) und der zweiten Nadel (5) hergestellt oder unterbrochen wird.

18. Verfahren nach Anspruch 17, **worin:** die relative Bewegung mindestens eine laterale Bewegung und/oder eine Rotationsbewegung ist oder einschließt.

19. Verfahren nach einem der Ansprüche 13 bis 18, **worin:** die Biopsienadel (1) mindestens zwei Nadelelektroden (4, 3) aufweist,
- von denen mindestens eine oder zwei galvanisch mit dem Konnektor (315) oder mit dem Messkabel verbunden ist/sind, wenn der Abschussteil (11) geladen ist,
und/oder
von denen die galvanische Verbindung zum Konnektor (315) oder zum Messkabel unterbrochen wird, wenn der Abschussteil (11) abgefeuert wird.

20. Verfahren nach einem der Ansprüche 13 bis 19, **worin:** der Konnektor (315) oder das Messkabel mit einem Impedanzmessgerät verbunden ist, das zur Messung von Bioimpedanz geeignet ist.

21. Verfahren nach Anspruch 20, **worin:** das Messgerät außerdem mit mindestens einer entfernten Elektrode verbunden ist, wie einer Elektrode, die an der Haut einer Person befestigt ist.

## Revendications

1. Pistolet de biopsie (30), comprenant :
- une poignée d'outil biopsique (6) sur laquelle a été fixée une aiguille de biopsie (100) comprenant une première aiguille (1), au moins une autre aiguille (5) pouvant être déplacée par rapport à la première aiguille (1) et une cavité de biopsie (7), ladite poignée d'outil biopsique (6) comprenant une pièce de tir (11) ou étant fixée à ladite pièce de tir (11) configurée de sorte à provoquer un mouvement relatif de la première aiguille (1) et d'au moins une autre aiguille (5) de sorte que leur mouvement relatif provoque le prélèvement d'un échantillon de biopsie dans la cavité de biopsie (7) ;
**dans lequel** :
le pistolet de biopsie (30) comprend en outre au moins un contact élastique (41, 42), tel qu'au moins un ressort de contact, permettant de créer un contact galvanique avec une aiguille-électrode de biopsie correspondante (4, 3) et relié à un connecteur (315) ou à un câble de mesure ;
et dans lequel ledit pistolet de biopsie (30) est configuré de sorte à :
i) relier galvaniquement au moins une électrode (4, 3) au connecteur (315) ou au câble de mesure lorsque la pièce de tir (11) est chargée, et/ou
ii) à couper la connexion galvanique entre au moins l'une des électrodes (4, 3) et le connecteur (315) ou le câble de mesure lors du déclenchement de la pièce de tir (11).

2. Pistolet de biopsie (30) selon la revendication 1, **dans lequel** : au moins un contact élastique (41, 42) est stationnaire et ledit pistolet de biopsie (30) est disposé de sorte à déplacer la première aiguille (1) et au moins une autre aiguille (5) l'une par rapport à l'autre en déplaçant une fixation correspondante (45, 46).

3. Pistolet de biopsie (30) selon la revendication 2, **dans lequel** : la connexion galvanique entre au moins une aiguille-électrode (4, 3) et le connecteur (315) est opérée sur la fixation (45, 46) ou sur chacune des fixations, respectivement.

4. Pistolet de biopsie (30) selon la revendication 3, **dans lequel** : la fixation (45) comprend un contact fixe (49) et le contact élastique correspondant (41) peut toucher le contact fixe (49) lorsque la pièce de tir (11) est chargée et/ou toucher le contact fixe (49) après le déclenchement de la pièce de tir (11) .

5. Pistolet de biopsie (30) selon l'une quelconque des revendications précédentes, **dans lequel** : la connexion galvanique entre au moins une aiguille-électrode (4, 3) et le connecteur (315) est établie sur la première aiguille (1).

6. Pistolet de biopsie (30) selon l'une quelconque des revendications précédentes, **dans lequel** : au moins un contact élastique (41, 42) est établi sur le couvercle dudit pistolet de biopsie (314).

7. Pistolet de biopsie (30) selon l'une quelconque des revendications précédentes, **dans lequel** : au moins un contact élastique (41, 42) est situé dans le réceptacle (40) d'une aiguille de biopsie (100).

8. Pistolet de biopsie (30) selon l'une quelconque des revendications précédentes, **dans lequel** : l'aiguille de biopsie (1) dispose au moins de deux aiguilles-électrodes (4, 3),
- dont l'une au moins ou les deux sont connectées au connecteur (315) ou au câble de mesure lorsque la pièce de tir (11) est chargée,
et/ou
- dont la connexion avec le connecteur (315) ou le câble de mesure est déconnectée lors du déclenchement de la pièce de tir (11).

9. Pistolet de biopsie (30) selon l'une quelconque des revendications précédentes, **dans lequel** :
- la première aiguille (1) et au moins une autre aiguille (5) ont une extrémité distale présentant une forme qui permet de couper les tissus ;
- l'aiguille-électrode de biopsie (4) comprend un fil-électrode (4) fonctionnant comme une électrode de mesure, ledit fil-électrode (4) s'étendant au-delà de la première aiguille (1) afin d'obtenir une zone de contact élargie permettant d'établir un contact galvanique avec un contact élastique (41, 42) du pistolet de biopsie (30).

10. Pistolet de biopsie (30) selon la revendication 9, **dans lequel** : le fil-électrode (4) est relié ou galvaniquement connecté à un contact fixe (49).

11. Système de prélèvement d'échantillons biopsiques **comprenant** : un pistolet de biopsie (30) selon l'une quelconque des revendications 1 à 10 et dans lequel le connecteur (315) ou le câble de mesure est connecté à un dispositif de mesure d'impédance permettant de mesurer la bio-impédance.

12. Système de prélèvement d'échantillons biopsiques selon la revendication 11, **dans lequel** : le dispositif de mesure d'impédance est en outre connecté à au moins une électrode située en dehors de l'aiguille de biopsie (100) de sorte qu'au moins une électrode est à distance d'au moins une aiguille-électrode (3, 4), ladite électrode étant de préférence collée à la peau de la personne.

13. Procédé pour connecter au moins une aiguille-électrode (4, 5) d'une aiguille de biopsie (100) à un connecteur (315) ou à un câble de mesure dans un pistolet de biopsie (30) selon l'une quelconque des revendications 1 à 10, ledit procédé **comprenant les étapes de** :
i) connexion galvanique d'au moins une aiguille-électrode (4, 3) au connecteur (315) ou au câble de mesure lorsque la pièce de tir (11) est chargée,
et/ou
ii) déconnexion de la connexion galvanique entre au moins une aiguille-électrode (4, 3) et le connecteur (315) ou le câble de mesure lors du déclenchement de la pièce de tir (11).

14. Procédé selon la revendication 13, **dans lequel** : la connexion et/ou la déconnexion sont effectuées en déplaçant au moins une fixation (45, 46) de l'aiguille de biopsie (100).

15. Procédé selon la revendication 13 ou la revendication 14, **dans lequel** : la connexion/déconnexion est réalisée en déplaçant le fil-électrode (4) vers/depuis un contact élastique (41, 42) qui dépasse du couvercle du pistolet de biopsie (314) en direction du réceptacle (40).

16. Procédé selon la revendication 15, **dans lequel** : le déplacement est ou comprend au moins un mouvement latéral et/ou rotatif.

17. Procédé selon l'une quelconque des revendications 13 à 16, **dans lequel** : la connexion galvanique est établie ou supprimée à la suite d'un mouvement relatif de la première aiguille (1) et de la seconde aiguille (5).

18. Procédé selon la revendication 17, **dans lequel** : le mouvement relatif est ou comprend au moins un mouvement latéral et/ou rotatif.

19. Procédé selon l'une quelconque des revendications 13 à 18, **dans lequel** : l'aiguille de biopsie (1) présente au moins deux aiguilles-électrodes (4, 3),
- dont l'une au moins ou les deux sont connectées au connecteur (315) ou au câble de mesure lorsque la pièce de tir (11) est chargée,
et/ou
- dont la connexion avec le connecteur (315) ou le câble de mesure est déconnectée lors du déclenchement de la pièce de tir (11) .

20. Procédé selon l'une quelconque des revendications 13 à 19, **dans lequel** : le connecteur (315) ou le câble de mesure est connecté à un dispositif de mesure d'impédance permettant de mesurer la bio-impédance.

21. Procédé selon la revendication 20, **dans lequel** : le dispositif de mesure d'impédance est en outre connecté à au moins une électrode à distance de sorte qu'une électrode est collée à la peau de la personne.
